# EUROPEAN PATENT APPLICATION

(11) **EP 0 877 093 A1**
(43) Date of publication of application: **11.11.1998**
(21) Application number: 98303627.8
(22) Date of filing: 08.05.1998
(51) Int. Cl.: C12Q 1/04, G01N 33/58, A61K 49/00, G01N 33/49, G01N 24/08

(54) **Medium for blood culture and method of detection and identification of microorganism**

(30) Priority: 08.05.1997 JP 118472/97; 27.08.1997 JP 231585/97
(71) Applicant: Nippon Sanso Corporation, Tokyo 105-0003 (JP)
(72) Inventor: Fujiike, Mikako, Minato-ku, Tokyo 105-0003, (JP); Maeda, Akihiko, Minato-ku, Tokyo 105-0003, (JP); Murakami, Yuji, Minato-ku, Tokyo 105-0003, (JP); Abe, Toshifumi, Minato-ku, Tokyo 105-0003, (JP); Miyashita, Shin, Minato-ku, Tokyo 105-0003, (JP); Seto, Haruo, Tokyo 192-0902 (JP); Furihata Kazuo, Tokyo 136-0076 (JP)
(74) Representative: Dean, John Paul

(57) **Abstract**

According to a method comprising the steps of culturing a sample in a medium comprising a carbon source having ¹³C, or fluorine for blood culture; measuring a chemical shift of the cultured medium; detecting a presence of a microorganism in the sample; and identifying a species of the microorganism, the microorganism in the sample, and in particular in blood specimen from septic patients, can be detected and identified in a short time.

## Description

### BACKGROUND OF THE INVENTION

### 1. FIELD OF THE INVENTION

This invention relates to a method of detection and identification of microorganisms and a medium therefor. This invention relates to a method of detection and identification of microorganisms by analyzing a chemical shift of nuclear magnetic resonance (hereinafter, referred to as NMR) of metabolites of the cultured microorganism.

This application is based on patent application No. Hei9231585 filed in Japan, the content of which is incorporated herein by reference.

### 2. DESCRIPTION OF RELATED ART

Tests for microorganisms consists of detection of a microorganism (confirmation of the presence of a microorganism) and the identification of the class of the microorganism.

Detection tests for microorganisms are conducted according to the following procedure. 2-5 ml of a sample (blood) is put into a sterilized bottle for culture of bacteria, and immediately after the inoculation, the sample in the bottle is put in an incubator and cultured at 30-37 °C. The culture is kept under observation once daily for 14 days until the growth of a microorganism is recognized. The growth of a microorganism is recognized by the turbidity of the medium, the formation of gas in the medium (the formation of gas bubbles by bacteria), the change of a pH of the medium (the formation of acids by bacteria), and the like. Subsequently, the microorganisms are identified according to the following procedure. In testing blood specimen from septic patients, after the septicemia-causing microorganism is found, Gram staining and morphological observation are conducted. From the result of the Gram staining, the bacterium is classified to Gram-positive or Gram-negative, and this data and the morphological features make classification (grouping) of the spieces of the bacterium possible.

Next, the sample is inoculated to a medium for culturing common bacteria (a non-selection medium) and cultured. In cases where several kinds of bacteria are present, the culturing is repeated until monomicrobic colonies appear on the medium. Then, the bacterium obtained from each colony is inoculated to a selection medium where other kinds of bacteria cannot grow, and the spieces of the bacterium is identified by the morphological and biochemical features.

The above conventional method of detection of microorganisms has a problem with requiring human handling to make daily observation from inoculation of samples to detection of microorganisms.

In addition, the conventional method of identification has problems as follows:
1) repeated culturing takes a long time;
2) automatic identification is difficult since the preparation of samples must be done by hand;
3) handling pathogenic microorganisms is time-consuming, and a person handling them may be exposed to the danger of contamination by the pathogenic microorganisms; and
4) visual assessment results in variation in identification.

### SUMMARY OF THE INVENTION

A medium for blood culture according to the present invention is characterized by comprising one carbon source selected form the group consisting of carbon sources having carbon atoms at least some of which are labeled with stable-isotope, and carbon sources having hydroxyl groups at least some of which are substituted with fluorine.

A method for detection and identification of a microorganism according to the present invention is characterized by comprising the steps of culturing a sample with a medium for blood culture comprising one of a carbon source having carbon atoms at least some of which are labeled with stable-isotope, and a carbon source having hydroxyl groups at least some of which are substituted with fluorine; measuring a chemical shift of the cultured medium; and detecting a presence of a microorganism in the sample and identifying a species of the microorganism.

The above method for detection and identification of a microorganism may be applied to various samples and tests, in particular, the method is effective for detection and identification of an aerobic microorganism, and is effective for testing blood, particularly, blood specimen from septic patients.

According to the present invention, a number of the culture can be decreased, resulting in savings of space for culture equipment. The present invention makes an automatic and laborsaving test system possible, and shortens the time required in a test system. The present invention leads to an objective judgment by means of a chemical shift chart. In addition, the handling time of a microorganism can be shortened, resulting in decreased danger of contamination by a pathogenic microorganism.

In the present invention, a chemical shift of ¹H-NMR or ¹H observing ¹³C-NMR is preferably measured, thereby shortening the time of the detection and identification of a microorganism because of the high sensitivity of these NMR techniques.

In addition, in the present invention, a differential spectrum between a chemical shift of ¹³C-NMR, ¹H-NMR, or ¹H observing ¹³C-NMR of the medium before the culturing and that of the medium after the culturing is preferably measured, shortening the time of the detection and identification of a microorganism.

### BRIEF DESCRIPTION OF THE DRAWINGS

Fig. 1 shows a chemical shift chart of ¹³C-NMR for a culture of *Escherichia coli.*

Fig. 2 shows a chemical shift chart of ¹³C-NMR for a culture of *Staphylococcus aureus.*

Fig. 3 shows a chemical shift chart of ¹³C-NMR for a culture of *Candida albicans.*

Fig. 4 shows a chemical shift chart of ¹³C-NMR for a culture of *Pseudomonas aeruginosa.*

Fig. 5 shows a chemical shift chart of ¹³C-NMR for a mixed culture of *Escherichia coli* and *Pseudomonas aeruginosa.*

Fig. 6 shows a chemical shift chart of ¹³C-NMR for a culture of *Bacteroides fragilis.*

Fig. 7 shows a chemical shift chart of ¹³C-NMR for a medium.

Fig. 8 shows a chemical shift chart of ¹³C-NMR for a culture of *Escherichia coli.*

Fig. 9 shows a chemical shift chart of ¹³C-NMR for fresh human blood culture.

Fig. 10 shows a chemical shift chart of ¹³C-NMR for a culture in which both fresh human blood and *Escherichia coli* are present.

Fig. 11 shows a chemical shift chart of ¹³C-NMR for a culture in which both fresh human blood and *Staphylococcus aureus* are present.

Fig. 12 shows a chemical shift chart of ¹³C-NMR for a culture in which both fresh human blood and *Staphylococcus epidermidis* are present.

Fig. 13 shows a chemical shift chart of ¹³C-NMR for a culture in which both fresh human blood and *Pseudomonas aeruginosa* are present.

Fig. 14 shows a chemical shift chart of ¹³C-NMR for a culture in which both fresh human blood and *Candida albicans* are present.

Fig. 15 shows a chemical shift chart of ¹H observing ¹³C-NMR for a culture of *Escherichia coli.*

Fig. 16 shows chemical shift charts of ¹³C-NMR for a medium before culturing and a medium after culturing *Escherichia coli,* and a differential spectrum therebetween.

### DESCRIPTION OF THE PREFERRED EMBODIMENTS

One embodiment of a medium for blood culture according to the subject invention is characterized by a medium comprising one carbon source having carbon atoms at least some of which are stable-isotopes. Here, a carbon source means an organic compound assimilated by a microorganism for its growth. As a carbon source, any sugars such as monosaccharides, oligosaccharides, and polysaccharides may be used. As monosaccharides, hexoses such as glucose, mannose, galactose, and fructose; or any kinds of deoxy sugars, or amino sugars may be used. Pentoses may be also used. Among them, 1-¹³C-glucose may be preferably used. When 1-¹³C-glucose is used as a labeled material, the more ¹³C enrichment glucose contains, the easier analysis by NMR becomes. However, it is not necessary that 100% of the carbon atoms in the glucose be substituted with ¹³C. Labeled glucose or labeled sugars may be added 0.01-10% by weight to the medium, preferably 0.05-5% by weight, and more preferably 0.1-3% by weight.

Another embodiment of a medium for blood culture according to the subject invention is characterized by a medium comprising a carbon source having hydroxyl groups at least some of which are substituted with fluorine. Fluorine may be detected by NMR analysis in the same manner as stable carbon isotope (¹³C).

As a medium for blood culture, a medium which contains 3-50 g of animal proteins, or 3-50 g of bovine (or calf's) brain exudate, or 3-50 g of bovine heart exudate, or 3-30 g of peptone per 1000 ml of the medium, and also contains 0.1-15 g of sodium chloride, 0.1-15 g of disodium phosphate, and ¹³C-labeled glucose, may be preferably used. Here, the amount of the ¹³C-labeled glucose may be 0.01-10% by weight, preferably 0.05-5% by weight, and more preferably 0.05-0.25% by weight. Brain exudate and heart exudate contain amino acids, vitamins, hormones, and the like, as well as proteins. By the addition of such nutrients, even if only a small amount of glucose is added or only a small amount of an initial microorganism is inoculated to the medium, the growth rate is increased and the 1-¹³C-glucose is assimilated effectively, resulting in the economical advantages with savings in the use of expensive isotope-labeled substances. In addition, a medium may be used which contains 3-50 g of peptone and 0.1-15 g of sodium chloride with respect to 1000 ml of the medium and to which ¹³C-labeled glucose is added. Here, the amount of the ¹³C-labeled glucose may be 0.01-10% by weight, preferably 0.05-5% by weight, and more preferably 0.05-0.25% by weight. As peptone, soy-peptone may be preferably used. Peptone is a general term for a mixture of non-crystalline polypeptides and oligopeptides having reduced molecular sizes formed by the hydrolysis of proteins. By the addition of such peptides, even if only a small amount of glucose is added or only a small amount of an initial microorganism is inoculated to the medium, the growth rate is increased and the 1-¹³C-glucose is assimilated effectively, resulting in the economical advantages with savings in the use of expensive isotope-labeled substances.

According to the present invention, the test is conducted as follows. A medium is put in a test tube or a bottle for blood culture, or a special NMR tube and sterilized. A sample is inoculated to this medium and cultured with shaking at a temperature at which the bacteria can grow (for example, 30-37 °C). The culturing while shaking provides conditions in which aerobes can grow but anaerobes cannot grow.

The culture may be conducted in an NMR tube. Since a regular NMR tube has a small diameter, by means of turning the tube over repeatedly at appropriate intervals, enough contact or mixturing of gas and liquid can be achieved. When the culturing is conducted in an NMR test tube, transfer of the culture is not required, resulting in a excellent efficiency and safety of the culture.

A fixed period of time (8-24 hours, for example) after the inoculation of a sample, a fixed volume (600-1000 µl, for example) of the culture is withdrawn and poured into an NMR tube. When the culture is conducted in a special NMR tube, this step can be omitted. As an internal standard, dioxan is poured (10-20 µl, for example) into the tube. A ¹³C or ¹⁹F chemical shift of a sample is measured by an NMR apparatus. The magnetic field intensity may be 90-270 MHz, for example. By a ¹³C or ¹⁹F chemical shift chart, the metabolites (organic acids, alcohols, and the like) are classified, and the detection (the confirmation of the presence of a microorganism) and the identification of the microorganism are achieved by reference with data base of the patterns of the metabolites.

The method for detection and identification of a microorganism according to the present invention can be applied to various samples, and especially, to a medium and a method for detecting septicemia-causing microorganisms. Since a bacterium detected in blood is likely to be a septicemia-causing bacterium, and the medication effective against the septicemia-causing microorganism is very important, the method of the present invention is effective for the diagnosis and the treatment of septicemia.

Needless to say, a blood sample of septicemia means that the blood sample is expected to have the possibility of septicemia.

A microorganism assimilates a carbon source (a carbon source labeled with stable-isotope, or a carbon source having hydroxyl groups substituted with fluorine) of a medium, and each microorganism produces different kinds and amounts of metabolites such as organic acids, alcohols, and the like. From a mixed sample consisting of a microorganism, a media, and metabolites, the metabolites can be analyzed easily and precisely by the measurement of NMR absorbence.

The present invention is characterized by making the detection and identification of a microorganism possible without being affected by biological components in blood or by components in the medium.

Since natural sugars and glucose originally contain ¹³C, natural metabolites of microorganisms contain ¹³C and have their ¹³C chemical shifts. However, due to the low level of natural ¹³C, the sensitivity of analysis for natural ¹³C is too low to be detected by measurement in a short time. When glucose and a sugar having carbon atoms labeled with ¹³C are used as a carbon source, the ¹³C content included in produced substance increases and NMR analysis becomes highly-sensitive, easy, and speedy.

When a carbon source having hydroxyl groups substituted with fluorine is used, the substances produced by a microorganism contain ¹⁹F, and NMR analysis with ¹⁹F as a probe becomes highly-sensitive, easy, and speedy.

The chemical shift of ¹H-NMR can be measured 50 times more sensitively than the chemical shift of ¹³C-NMR, and the chemical shift of ¹H observing ¹³C-NMR can be measured 4 times more sensitively than the chemical shift of ¹³C-NMR. Therefore, in ¹H-NMR or ¹H observing ¹³C-NMR, the culturing time can be shortened to a half or a quarter of that of ¹³C-NMR.

When a differential spectrum between the chemical shift of ¹³C-, ¹H-, or ¹H observing ¹³C-NMR of the medium before culturing and that of the medium after culturing is measured, peaks of compounds which do not change before and after the culturing can be canceled, and as a result, only compounds which are consumed or generated in the culturing can be detected with high sensitivity.

### [Example 1: Independent culture of Each Strain]

As strains for the following tests, *Escherichia coli* ATCC29522, *Staphylococcus aureus* ATCC29213, *Candida albicans* ATCC90029, *Pseudomonas aeruginosa* ATCC28753 were used. These strains are aerobic and representative microorganisms which cause septicemia.

1% by weight of sterilized glucose-1-¹³C (stable carbon isotope concentration of 99%, manufactured by U.S. ISOTEC Company; this is to be also used in the following examples) was added to heart-infusion medium (manufactured by EIKEN CHEMICAL CO.,LTD; this is to be also used in the following examples).

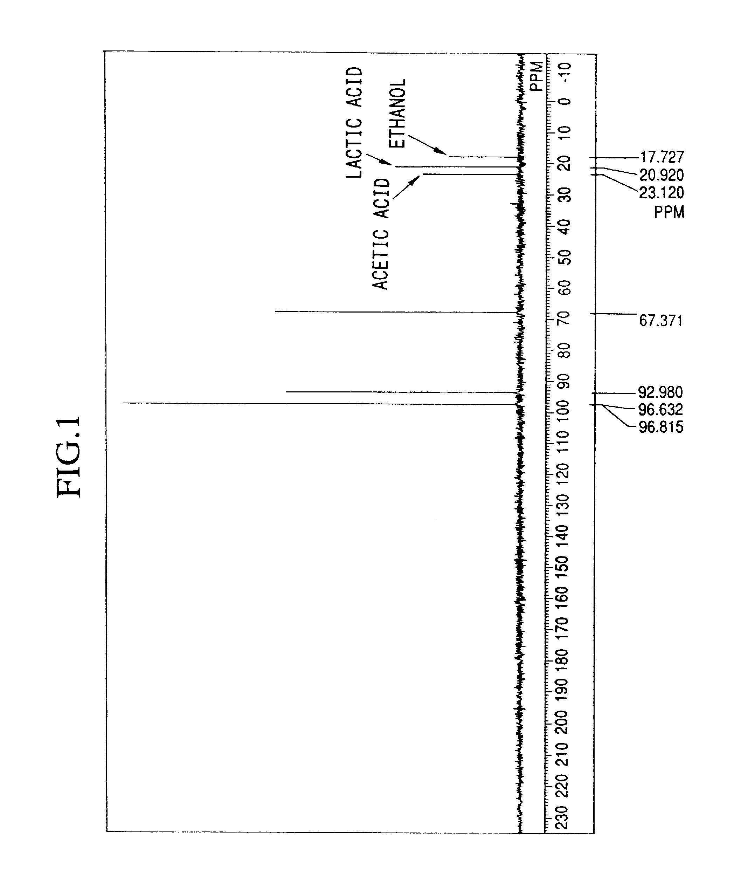

The above strains were inoculated to the medium (10⁵/ml) and cultured in an incubator at 35 °C for 24 hours with rotating (150 rpm) and shaking. 784 µl of the culture was withdrawn in an NMR tube with a diameter of 5 mm. Then, 16 µl of 1,4 -dioxan (special grade, manufactured by JUNSEI CHEMICAL CO.,LTD; this is to be also used in the following examples) was added. ¹³C- nuclear magnetic resonance of this sample was measured (apparatus: NMRGSX-270, manufactured by Japan Electron K.K.; with a magnetic field intensity of 270 MHz; this is to be also used in the following examples). The measurement of the sequence was conducted by the method of proton complete decoupling. The obtained chemical shift charts are shown in Figs. 1-4.

Fig. 1 is an NMR chemical shift chart of the medium in which *Escherichia coli* ATCC29522 was cultured. For *Escherichia coli,* it is characteristic that peaks of ethanol (17.727 ppm), lactic acid (20.920 ppm), and acetic acid (23.120 ppm) appear. In this chart, the peaks of 92.980 ppm, 96.632 ppm, and 96.815 ppm correspond to glucose, and the peak of 67.371 ppm corresponds to dioxan as an internal standard. Here, the locations of the peaks of the products in the chemical shift chart shift depending on the condition of the analysis, and specially, the peak of carbonic acid shifts considerably depending on the pH of the cultured medium (the same thing also happens in the following charts).

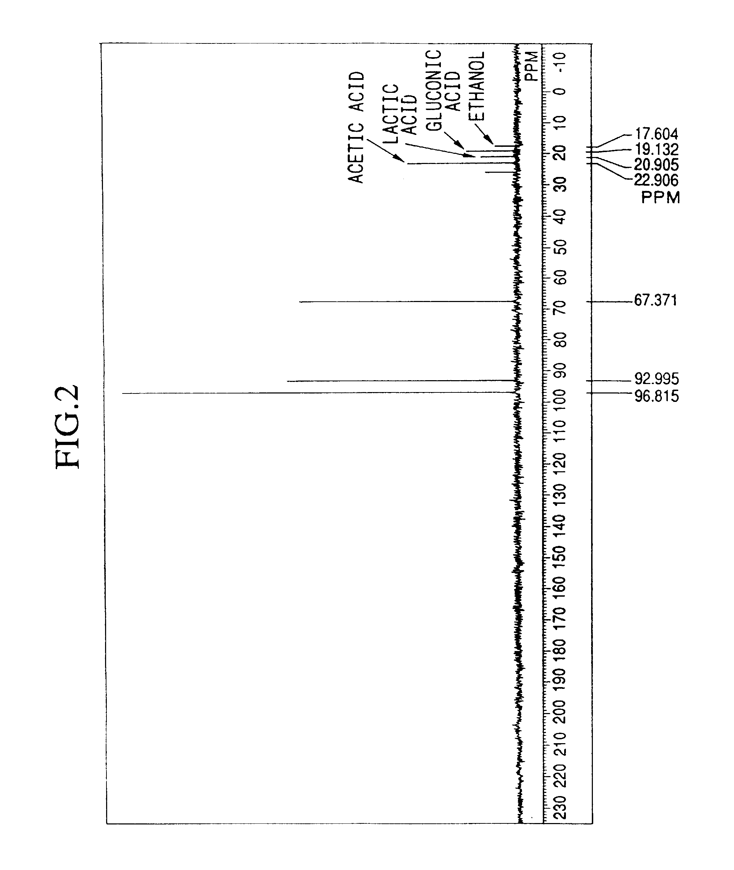

Fig. 2 is an NMR chemical shift chart of the medium in which *Staphylococcus aureus* ATCC29213 was cultured. For *Staphylococcus,* it is characteristic that peaks of ethanol (17.604 ppm), gluconic acid (19.132 ppm), lactic acid (20.905 ppm), and acetic acid (22.906 ppm) appear. In this chart, the peaks of 92.995 ppm and 96.815 ppm correspond to glucose, and the peak of 67.371 ppm corresponds to dioxan as an internal standard.

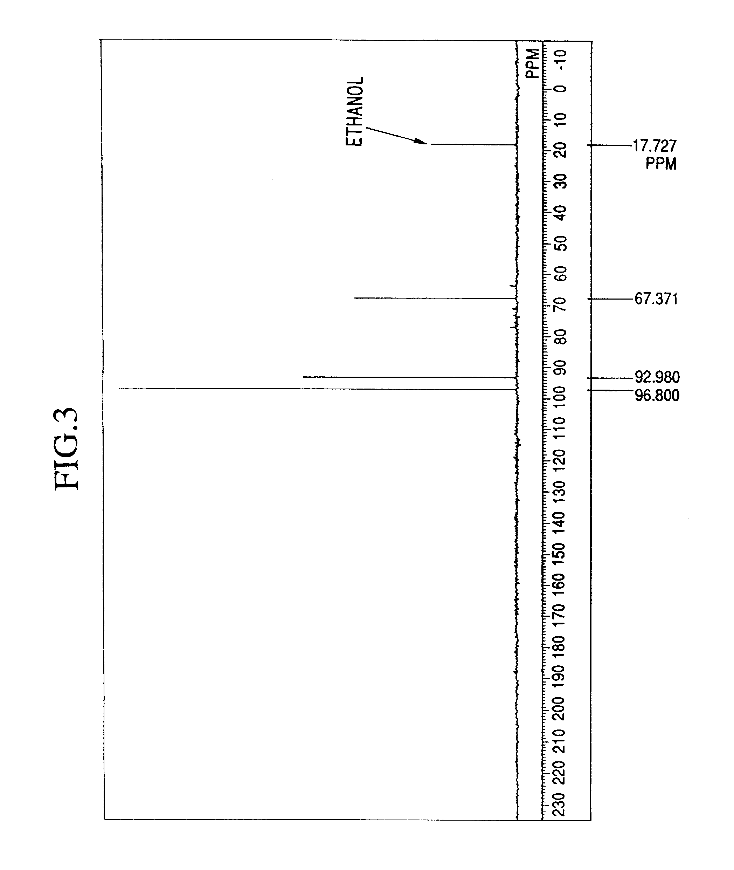

Fig. 3 is an NMR chemical shift chart of the medium in which *Candida albicans* ATCC90029 was cultured. For *Candida,* it is characteristic that peak of ethanol (17.727 ppm) appears. In this chart, peaks of 92.980 ppm and 96.800 ppm correspond to glucose, and the peak of 67.371 ppm corresponds to dioxan as an internal standard.

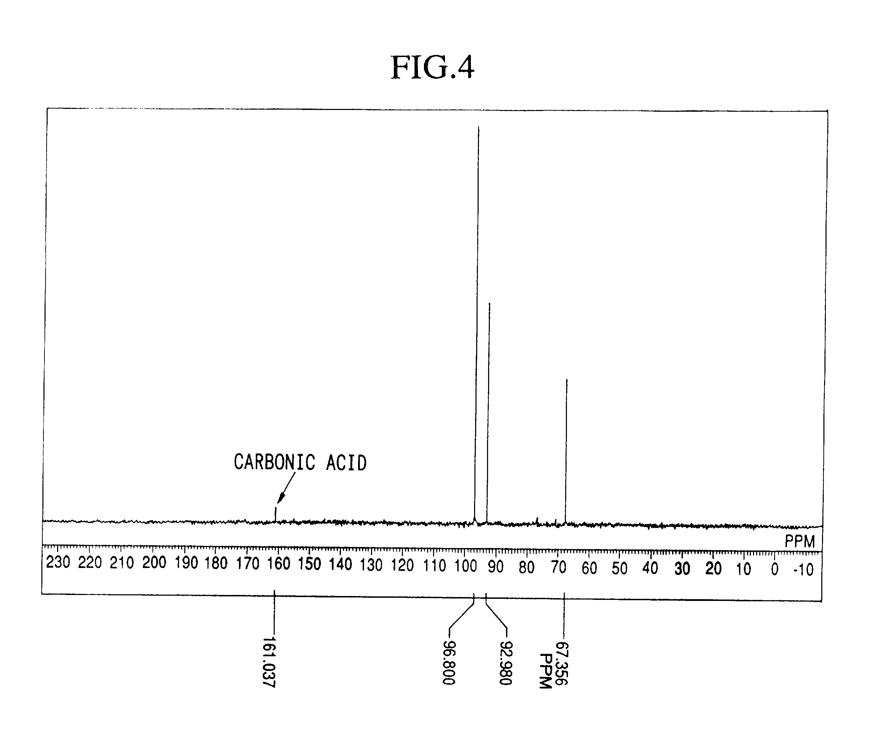

Fig. 4 is a NMR chemical shift chart of the medium in which *Pseudomonas aeruginosa* ATCC28753 was cultured. For *Pseudomonas,* it is characteristic that peak of carbonic acid (161.037 ppm) appears. Carbonic acid is carbon dioxide dissolved in water. In this chart, the peaks of 92.980 ppm and 96.800 ppm correspond to glucose, and the peak of 67.356 ppm corresponds to dioxan as an internal standard.

Since the chemical shift charts of the above four strains are characteristic and distinguishable from each other as described above, they make the detection and identification of strains possible.

### [Example 2: Mixed Culture of Two Strains]

As strains for the following tests, *Escherichia coli* ATCC29522 and *Pseudomonas aeruginosa* ATCC28753 were used. These strains are aerobic and representative microorganisms which cause septicemia, as described above.

1% by weight of sterilized glucose-1-¹³C was added to heart-infusion medium.

*Escherichia coli* ATCC29522 (0.9 x 10⁵/ml) and *Pseudomonas aeruginosa* ATCC28753 (0.1 x 10⁵/ml) were inoculated to the medium and cultured in an incubator at 35 °C for 24 hours with rotating (150 rpm) and shaking. 784 µl of the culture was withdrawn in an NMR tube with a diameter of 5 mm. Then, 16 µl of 1,4 -dioxan was added. ¹³C-nuclear magnetic resonance of this sample was measured. The measurement of the sequence was conducted by the method of proton complete decoupling.

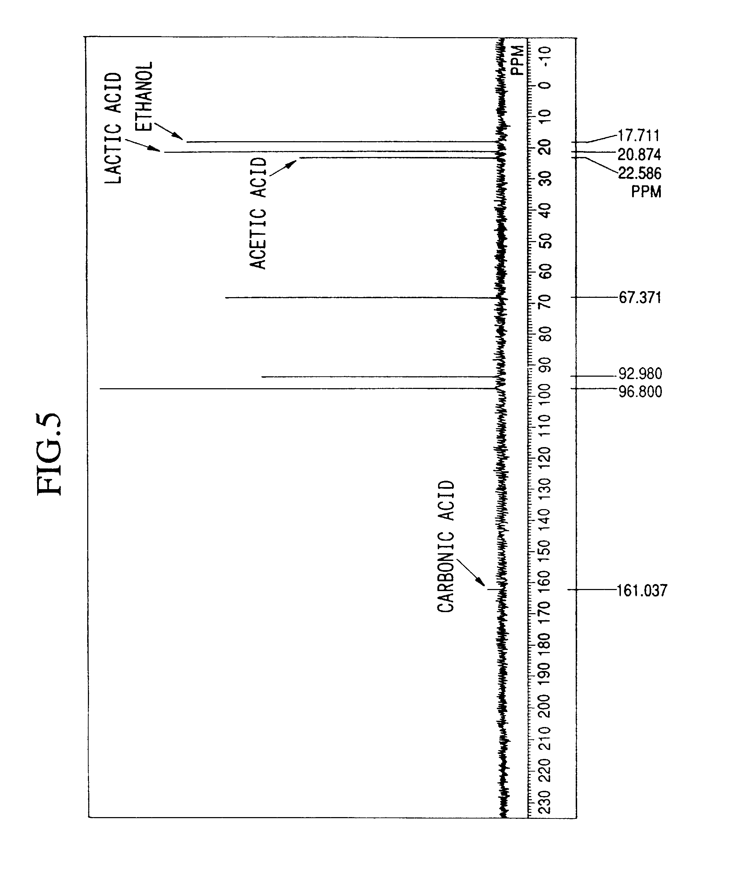

The obtained chemical shift chart is shown in Fig. 5. The peak of 17.711 ppm corresponds to ethanol, the peak of 20.874 ppm corresponds to lactic acid, and the peak of 22.586 ppm corresponds to acetic acid. By comparison between this chart and the chart of Example 1, the presence of *Escherichia coli* in this sample is shown. The peak of 17.711 ppm corresponds to ethanol, the peak of 20.874 ppm corresponds to lactic acid, and the peak of 22.586 ppm corresponds to acetic acid. By comparison between this chart and the chart of Example 1, the presence of *Escherichia coli* in this sample is shown. The peak of 161.037 ppm corresponds to carbonic acid. By comparison between this chart and the chart of Example 1, the presence of *Pseudomonas aeruginosa* in this sample is shown. The peak of 96.800 ppm corresponds to glucose and the peak of 67.371 ppm corresponds to dioxan as an internal standard.

As described above, a comparison of patterns between the NMR chart of mixed culture of two strains and the chart obtained by Example 1 shows the coexistence of *Escherichia coli* ATCC29522 and *Pseudomonas aeruginosa* ATCC28753 in the sample of Example 2.

### [Comparative Example 1: An Anaerobic Strain]

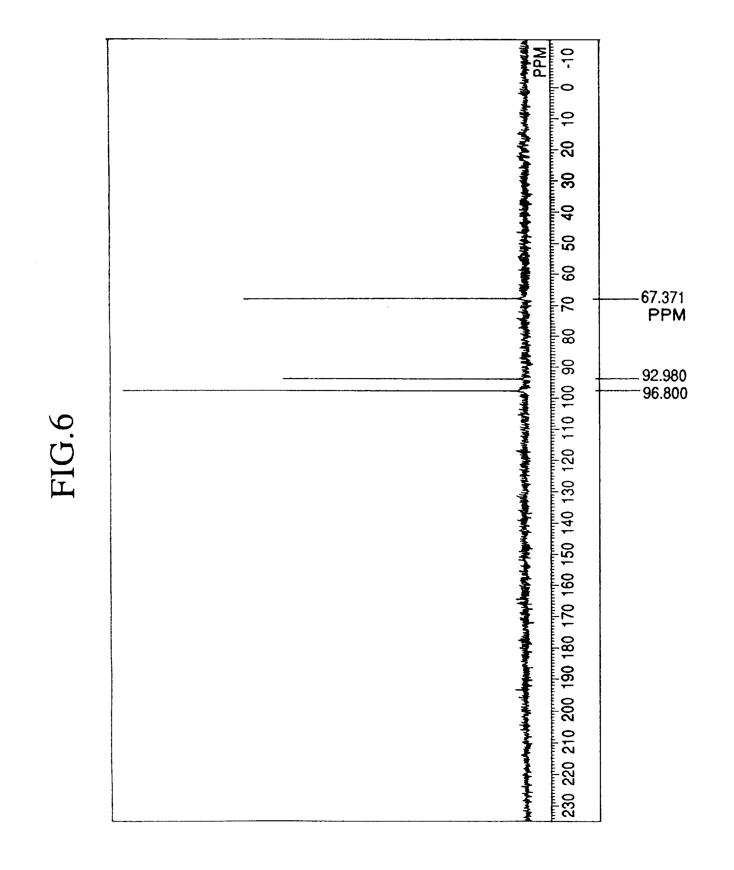

The culturing was conducted in an aerobic condition with shaking in the same manner as Example 1, except that *Bacteroides fragilis,* which is an anaerobic bacterium, was used. The chemical shift chart of the culture is shown in Fig. 6. The peaks of 92.980 ppm and 96.800 ppm correspond to glucose, and the peak of 97.371 ppm corresponds to dioxan as an internal standard. Any Other peak characteristic to *Bacteroides* was not found in this chart, that is, an anaerobic microorganism was not confirmed in this example.

### [Example 3: Cultures of Microorganism in Blood]

The purpose of this example is to prove the possibility of the detection and identification of the microorganism when the microorganism and blood were cultured together in a medium labeled with stable isotope according to the present invention.

As blood, fresh blood collected from healthy people was used.

As strains, five strains, *Escherichia coli* ATCC29522, *Staphylococcus aureus* ATCC29213, *Staphylococcus epidermidis* ATCC12228, *Pseudomonas aeruginosa* ATCC28753, and *Candida albicans* ATCC90029 were used.

As a medium, brain-heart-infusion medium was used. This medium contained 12.5 g of calf's brain exudate, 5 g of bovine heart exudate, 10 g of peptone, 1.5 g (0.15% by weight) of glucose-1-¹³C, and also contained 2.5 g of sodium chloride, and 2.5 g of disodium phosphate, per 1000ml of the medium.

1 ml of fresh human blood was added to 9 ml of the sterilized medium in a test tube. The above strains were inoculated to the medium respectively (at two grades of the inoculation size: 10² /ml and 10⁵/ml) and cultured in an incubator at 37 °C for 16 hours with rotating at 70 rpm. A control sample with fresh human blood added and no microorganism inoculated, was cultured in the same condition as above. After the culture for 16 hours, 784 µl of the culture was withdrawn and put in an NMR tube with a diameter of 5 mm. Then, 16 µl of 1,4 -dioxan was added. ¹³C- nuclear magnetic resonance of this sample was measured. The measurement of the sequence was conducted by the method of proton complete decoupling.

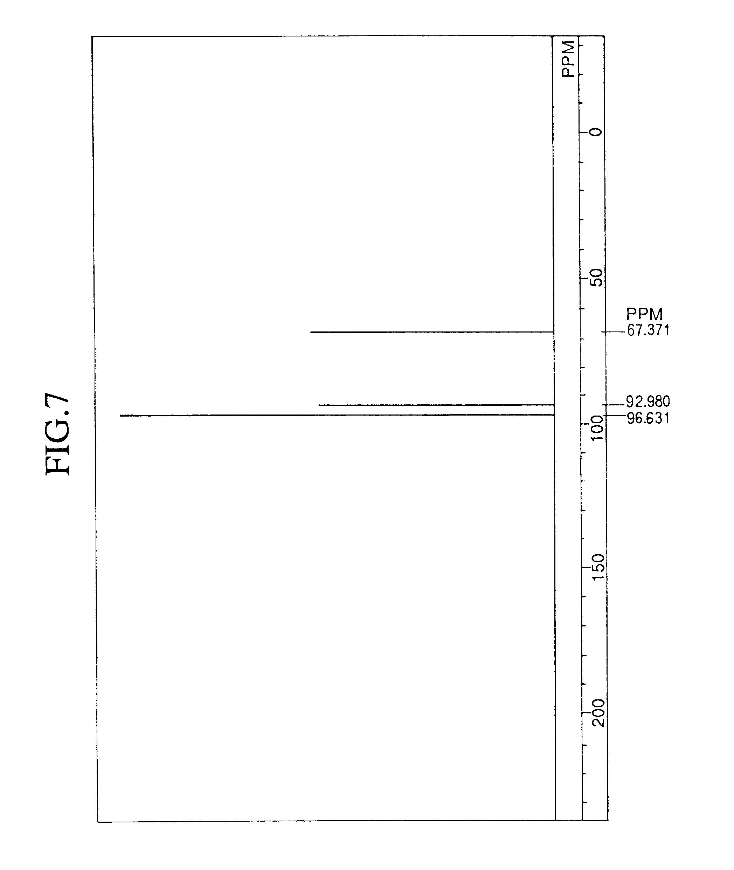

Fig. 7 shows a chemical shift chart of ¹³C-NMR of the control (the medium only).

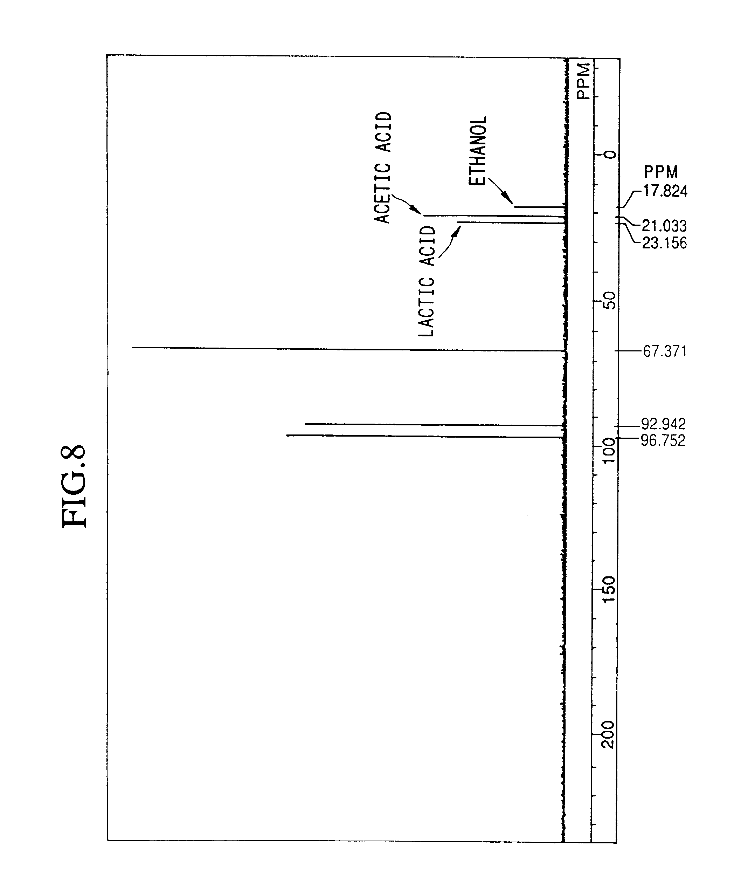

Fig. 8 shows a chemical shift chart of ¹³C-NMR of the culture medium after only *Escherichia coli* ATCC29522 was inoculated to the medium (the bacterial inoculation of 10⁵/ml) and cultured. There are the peaks of ethanol at 17.824 ppm, of lactic acid at 21.033 ppm, and of acetic acid at 23.156 ppm.

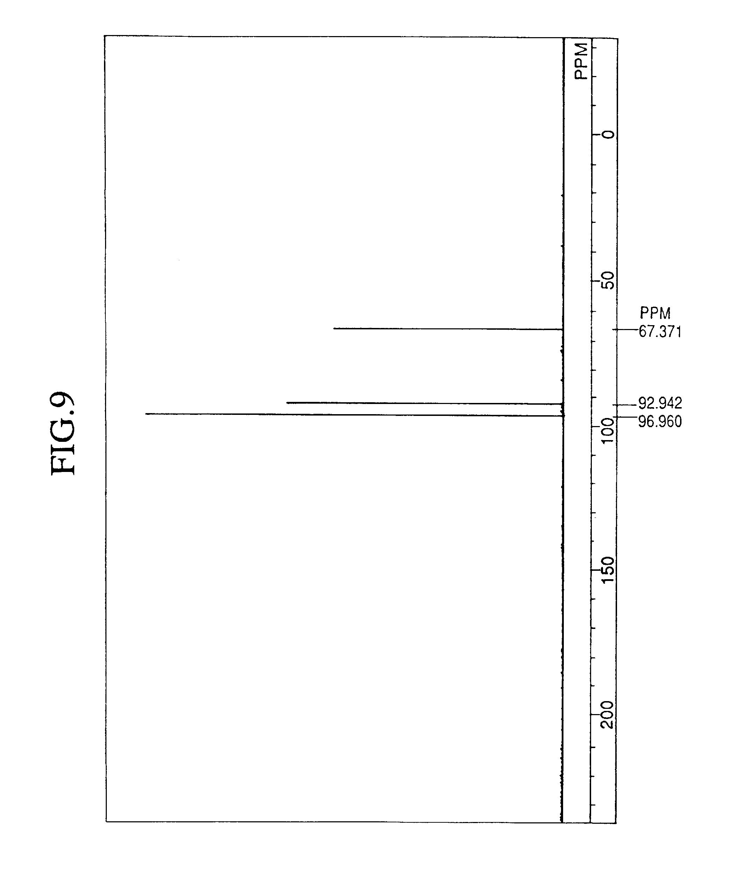

Fig. 9 shows a chemical shift chart of ¹³C-NMR of the culture of fresh human blood only. In this chart, there are no peaks of ethanol, lactic acid, or acetic acid.

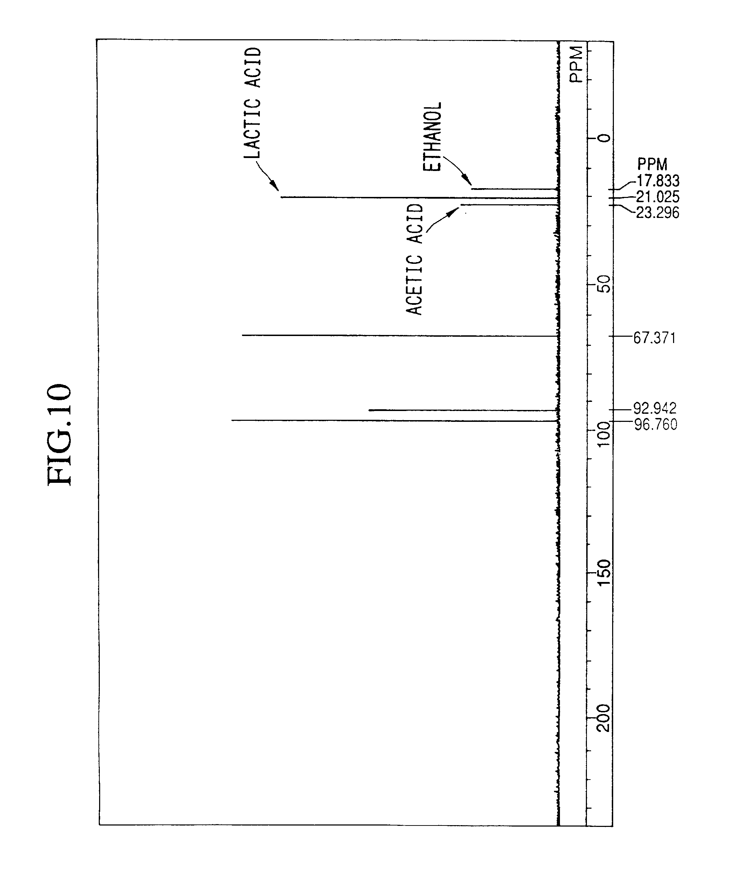

Fig. 10 shows a chemical shift chart of ¹³C-NMR for culture in which both fresh human blood and *Escherichia coli* are present. In this chart, there are the peaks of ethanol, lactic acid, and acetic acid at almost the same positions as in Fig. 8.

As described above, in the culture in which both fresh human blood and *Escherichia coli* are present, the same chemical shift chart can be obtained as in an independent culture of *Escherichia coli* without being affected by the components of blood. Thus, according to the present invention, culturing, detection, and identification of a microorganism in blood is possible.

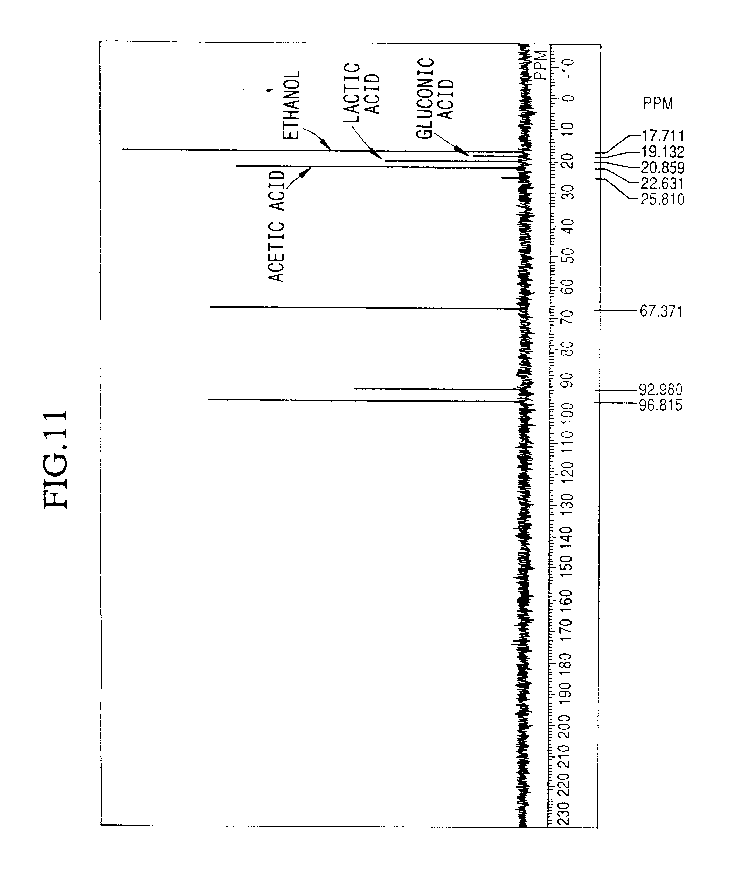

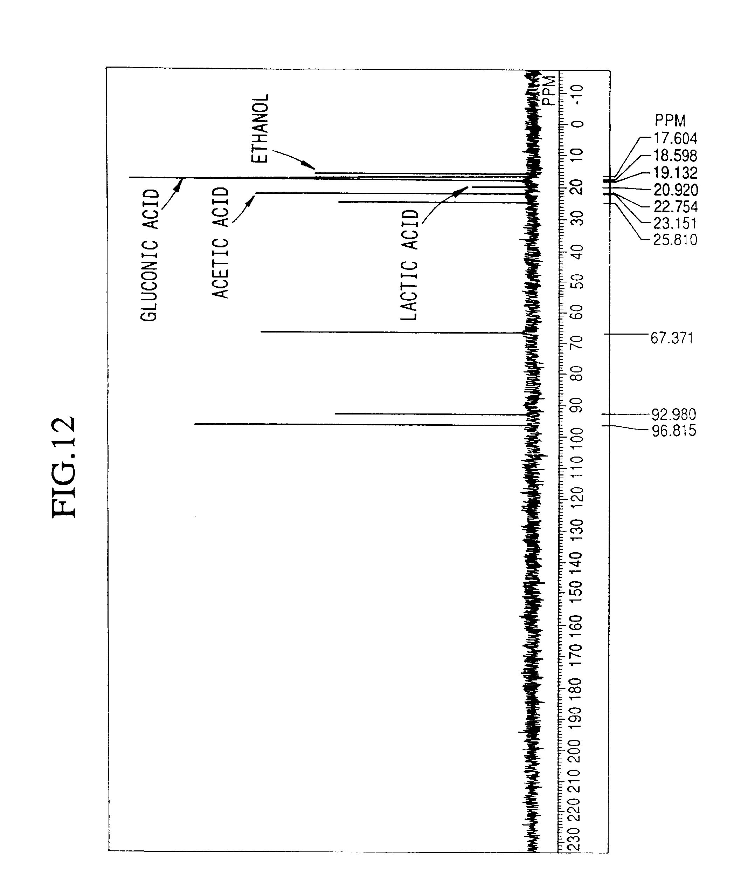

Fig. 11 shows a chemical shift chart of ¹³C-NMR for a culture in which both fresh human blood and *Staphylococcus aureus* ATCC29213 are present. Fig. 12 shows a chemical shift chart of ¹³C-NMR for a culture in which both fresh human blood and *Staphylococcus epidermidis* ATCC 12228 are present. The culture in which fresh human blood and *Staphylococcus epidermidis* are present, can be distinguished from the culture in which fresh human blood and *Staphylococcus aureus* are present, by the appearance of a peak next to the peak of gluconic acid and by the difference in height of the peaks of the metabolites.

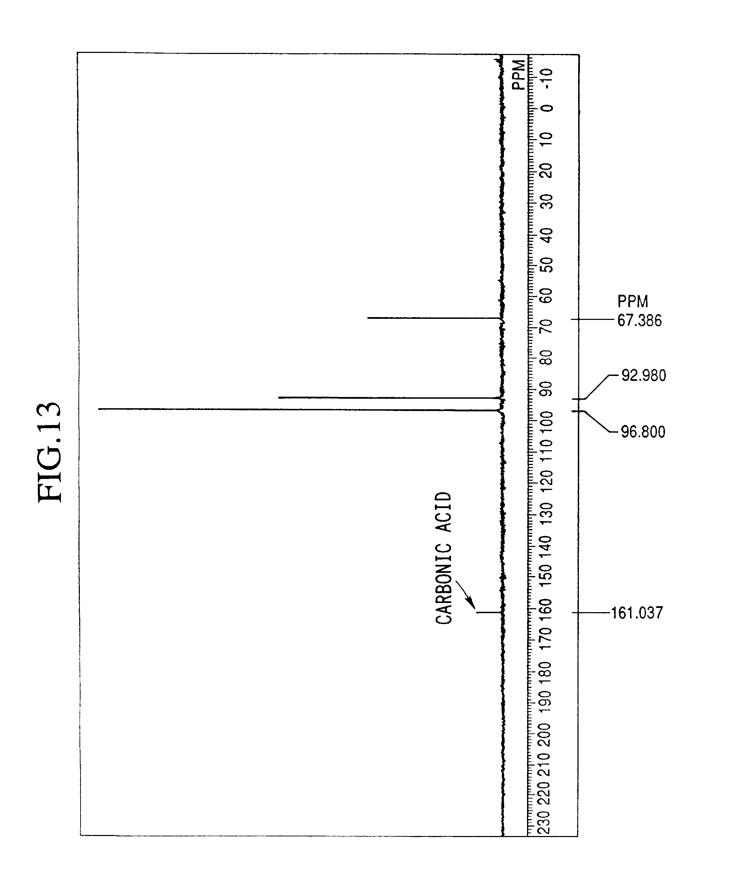

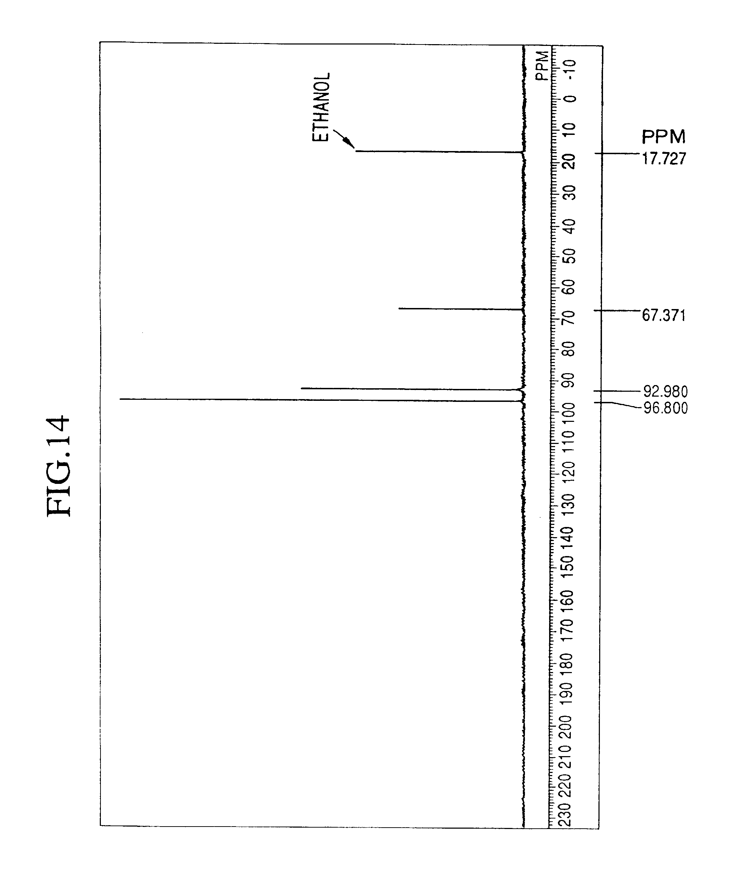

Fig. 13 shows a chemical shift chart of ¹³C-NMR for a culture in which both fresh human blood and *Pseudmonas aeruginosa* ATCC28753 are present. Fig. 14 shows a chemical shift chart of ¹³C-NMR for a culture in which fresh human blood and *Candida albicans* ATCC90029 are present.

As shown in Example 1, the chemical shift charts of the above strains are distinguishable from each other by the characteristic metabolites and the height of the peaks.

In the above, cultures with an inoculated concentration of 10⁵/ml were discussed. Similarly, at the inoculated concentrations of 10²/ml, culturing, detection, and identification of the microorganism in blood was also possible.

As described in the above Examples, detection and identification of strains were possible for cultures in which fresh human blood and the microorganism were present, that is, in cultures of the microorganism in blood, without being affected by the components of blood.

### [Example 4: ¹H observing ¹³C-NMR]

This example is to show the possibility of the reduction of the culturing time required to detect a microorganism due to the high sensitivity of the ¹H observing ¹³C-NMR. As a strain for the following test, *Escherichia coli* ATCC29522 was used.

1% by weight of sterilized D-glucose-1-¹³C was added to sterilized heart-infusion medium. The above strain was inoculated to the medium (10⁵/ml) and cultured in an incubator at 35 °C for 4 hours with rotating (150 rpm) and shaking. 784 µl of the culture was withdrawn in an NMR tube with a diameter of 5 mm. Then, an NMR of this sample was analyzed. The measurement of the sequence was conducted by the method of heteronuclear single-quantum coherence.

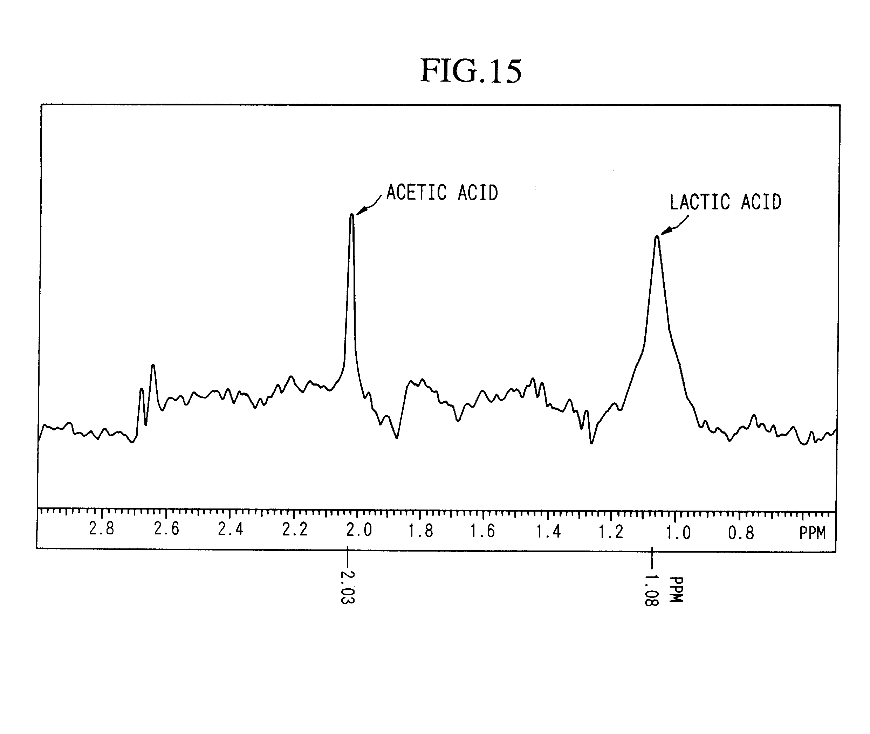

The obtained chemical shift chart is shown in Fig. 15. The peak of lactic acid at about 1.08 ppm and the peak of acetic acid at about 2.03 ppm are observed, these compounds being metabolites of *Escherichia coli.* Thus, the strain can be detected even with a remarkably decreased time of culturing compared with Example 1.

### [Example 5: Differential Spectrum between Medium before Culturing and Medium after Culturing]

In order to show the possibility of reducing the culturing time required to detect a microorganism by measuring a differential spectrum of a chemical shift of a medium between before culturing and a medium after culturing, the following test was conducted. As a test strain, *Escherichia coli* ATCC29522 was used.

1% by weight of sterilized D-glucose-1-¹³C was added to sterilized heart-infusion medium. The above strain was inoculated to the medium (10⁵/ml) and cultured in an incubator at 35 °C for 4 hours with rotating (150 rpm) and shaking. 784 µl of the culture was withdrawn in an NMR tube with a diameter of 5 mm, and 16 µl of 1,4 -dioxan was added. Then, an NMR of this sample was analyzed. The measurement of the sequence was conducted by the method of proton complete decoupling.

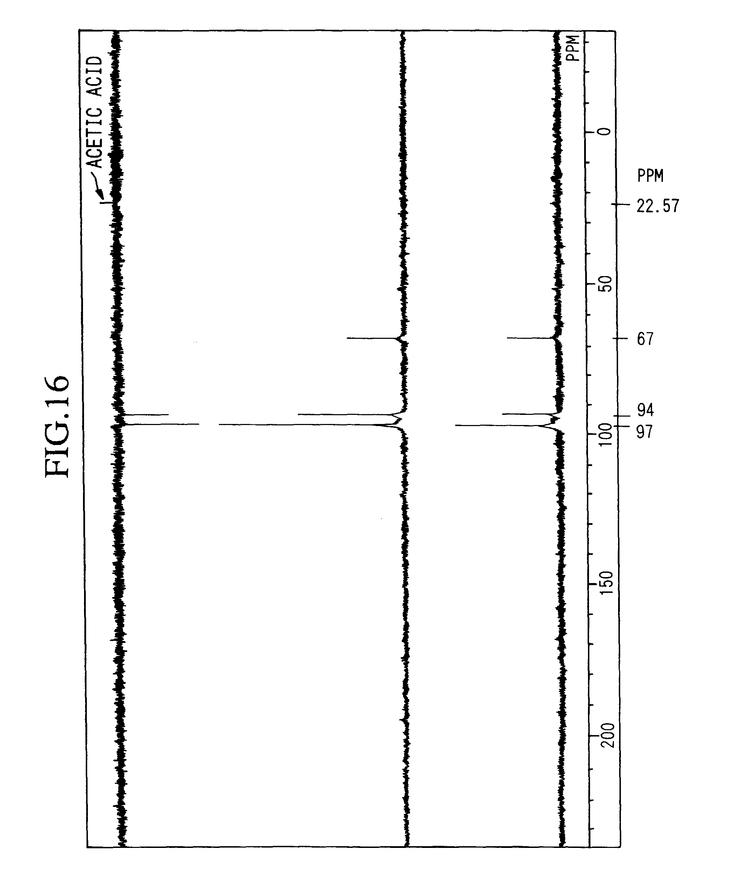

The obtained chemical shift charts are shown in Fig. 16. In Fig. 16, the middle chart shows a spectrum of the medium before the culturing, the bottom chart shows a spectrum of the medium after the culturing, and the upper chart shows a differential spectrum therebetween. The peak of acetic acid, a metabolite of *Escherichia coli,* is clearly observed at about 22.57 ppm in the upper chart, although this peak can hardly be distinguished from noise in the bottom chart. In this way, by means of a differential spectrum, the sensitivity of the detection is increased, and a strain can be detected even when the time of the culturing is shortened. Here, the peaks at about 94 ppm and about 97 ppm correspond to glucose, and a peak at about 67 ppm corresponds to dioxan.

## Claims

1. A medium for blood culture comprising a carbon source having at least one carbon atom labelled with a stable-isotope or a carbon source having at least one hydroxyl group substituted with fluorine.

2. A medium for blood culture according to claim 1, characterised in that the carbon source is a sugar labelled with a stable-isotope.

3. A medium for blood culture according to claim 1 or claim 2, characterised in that the carbon source is 1-¹³C-glucose labelled with a stable-isotope.

4. A medium for blood culture according to any one of claims I to 3, characterised in that the medium contains an animal protein.

5. A medium for blood culture according to any one of claims 1 to 4, characterised in the medium further contains bovine brain exudate or bovine heart exudate.

6. A medium for blood culture according to any one of claims 1 to 5, characterised in that the medium further contains peptone.

7. A method for detection and identification of a microorganism comprising the steps of:
culturing a sample with a medium for blood culture according to any preceding claim;
measuring a chemical shift of the cultured medium; and
detecting the presence of a microorganism in the sample and identifying the species of the microorganism.

8. A method for detection and identification of a microorganism according to claim 7, characterised in that the chemical shift is a chemical shift of ¹³C-nuclear magnetic resonance.

9. A method according to claim 7, characterised in that the chemical shift is a chemical shift of 'H-nuclear magnetic resonance or of 'H observing ¹³C-nuclear magnetic resonance.

10. A method according to any one of claims 7 to 9, characterised in that the microorganism causes septicemia.

11. A method according to claim 10, characterised in that the microorganism is an aerobe.

12. A method according to any one of claims 7 to 11, characterised in that the sample is blood to be tested for the presence of a septicemia-causing microorganism.

13. A method according to any one of claims 7 to 12, in which a chemical shift of the nuclear magnetic resonance indicating the presence of ethanol, lactic acid, and acetic acid indicates the presence of *Escherichia coli.*

14. A method according to any one of claims 7 to 12, in which a chemical shift of the nuclear magnetic resonance indicating the presence of ethanol, gluconic acid, lactic acid, and acetic acid indicates the presence of *Staphylococcus spp.*

15. A method according to any one of claims 7 to 12, in which a chemical shift of the nuclear magnetic resonance indicating the presence of ethanol indicates the presence of *Candida sp.*

16. A method according to any one of claims 7 to 12, in which a chemical shift of the nuclear magnetic resonance indicating the presence of carbonic acid indicates the presence of *Pseudomonas sp.*

17. A method for the detection and identification of strains according to any one of claims 7 to 16, characterised in that the sample is cultured with the medium in a nuclear magnetic resonance test tube.

18. A method for the detection and identification of a microorganism comprising the steps of:
culturing a sample with a medium for blood culture according to any one of claims 1 to 6;
measuring a differential spectrum between a chemical shift of the medium before the culturing and a chemical shift of the medium after the culturing; and
detecting the presence of a microorganism in the sample and identifying the species of the microorganism by the differential spectrum.

19. A method for detection and identification of a microorganism according to claim 18, characterised in that the chemical shift is a chemical shift of ¹³C-nuclear magnetic resonance.

20. A method according to claim 17, wherein the chemical shift is a chemical shift of 'H-nuclear magnetic resonance or of 'H observing ¹³C-nuclear magnetic resonance.
